# EUROPÄISCHE PATENTSCHRIFT

(11) **EP 3 049 071 B1**
(45) Veröffentlichungstag und Bekanntmachung des Hinweises auf die Patenterteilung: **17.11.2021**
(21) Anmeldenummer: 14777049.9
(22) Anmeldetag: 25.09.2014
(51) Int. Cl.: A61K 31/00, A61K 31/4166, A61K 31/517, A61P 25/28

(54) **PROPHYLAXE UND BEHANDLUNG EINER NICHT AUF EINER PROTEINFALTUNGSSTÖRUNG BERUHENDEN NEURODEGENERATIVEN ERKRANKUNG**
PROPHYLAXIS AND TREATMENT OF A NEURODEGENERATIVE DISEASE NOT BASED ON A PROTEIN FOLDING DISORDER
PROPHYLAXIE ET TRAITEMENT D'UNE MALADIE NEURODÉGÉNÉRATIVE NON DUE À UN TROUBLE DU REPLIEMENT DES PROTÉINES

(30) Priorität: 27.09.2013 DE 102013110714
(43) Veröffentlichungstag der Anmeldung: 03.08.2016
(73) Patentinhaber: Eberhard Karls Universität Tübingen Medizinische Fakultät, 72074 Tübingen (DE)
(72) Erfinder: UEFFING, Marius, 72076 Tübingen (DE); ARANGO-GONZALEZ, Blanca, 72764 Reutlingen (DE); GRICIUC, Ana, Somerville, Massachusetts 02144 (US)
(74) Vertreter: Witte, Weller & Partner Patentanwälte mbB
(86) Internationale Anmeldenummer: PCT/EP2014/070459
(87) Internationale Veröffentlichungsnummer: WO 2015/044251

(56) Entgegenhaltungen:
- EP-A1- 2 623 494
- WO-A2-2009/011910
- Anonymous: "B6.CXB1-Pde6brd10/J", The Jackson Laboratory , 1. Januar 2014 (2014-01-01), XP002732290, Gefunden im Internet: URL:http://jaxmice.jax.org/strain/004297.h tml [gefunden am 2014-11-10]
- TSUI-FEN CHOU ET AL: "Structure-Activity Relationship Study Reveals ML240 and ML241 as Potent and Selective Inhibitors of p97 ATPase", CHEMMEDCHEM, Bd. 8, Nr. 2, 11. Januar 2013 (2013-01-11) , Seiten 297-312, XP055152147, DE ISSN: 1860-7179, DOI: 10.1002/cmdc.201200520
- LAURA CAMPELLO ET AL: "The Ubiquitin-Proteasome System in Retinal Health and Disease", MOLECULAR NEUROBIOLOGY, vol. 47, no. 2, 22 January 2013 (2013-01-22), pages 790-810, XP055470981, US ISSN: 0893-7648, DOI: 10.1007/s12035-012-8391-5
- Shang Fu ET AL: "Function of the ubiquitin proteolytic pathway in the eye", EXPERIMENTAL EYE RESEARCH., vol. 78, no. 1, 1 January 2004 (2004-01-01), pages 1-14, XP055805470, LONDON. ISSN: 0014-4835, DOI: 10.1016/j.exer.2003.10.003

## Beschreibung

Die vorliegende Erfindung betrifft Substanzen zur Prophylaxe und Behandlung einer nicht auf einer Proteinfaltungsstörung beruhenden neurodegenerativen Erkrankung.

Neurodegenerative Erkrankungen bilden eine Gruppe von meist langsam fortschreitenden, erblich oder sporadisch auftretenden Erkrankungen des Nervensystems. Hauptmerkmal ist der zunehmende Verlust von Nervenzellen, der zu verschiedenen neurologischen Symptomen führt, darunter häufig zu Demenz und Bewegungsstörungen. Die Erkrankungen können in unterschiedlichen Lebensaltern auftreten, verlaufen diffus oder generalisiert und rufen charakteristische Schädigungsmuster hervor.

Von besonderer Bedeutung sind neurodegenerative Erkrankungen des Auges.

Die retinale Degeneration ist ein Verfall der Retina, der schließlich zum Tod der Netzhautzellen führen kann. Eine der wichtigsten Formen der Retinadegeneration ist die sogenannte Retinitis pigmentosa (RP) oder auch Retinopathia pigmentosa genannt. Weltweit sind von RP etwa 3 Millionen Menschen, in Deutschland etwa 30.000 bis 40.000, betroffen. Die Erkrankung tritt meistens im Jugendalter oder in den mittleren Lebensjahren mit den ersten Merkmalen, wie beispielsweise Nachtblindheit, auf. Die Sehkraft lässt allmählich nach. Der gesamte Prozess zunehmender Sehbehinderung verläuft schleichend und erstreckt sich beim Betroffenen meistens über Jahrzehnte hinweg.

Unter dem Begriff Maculadegeneration wird eine Gruppe von neurodegenerativen Erkrankungen des Auges zusammengefasst, die die Macula lutea, auch "gelber Fleck" genannt, der Netzhaut betreffen und mit einem allmählichen Funktionsverlust der dort befindlichen Gewebe einhergehen. Die wichtigste Form bildet hier die altersbedingte Makuladegeneration (AMD) in ihrer trockenen oder feuchten Form. Sie beginnt durch Ablagerungen von sogenannten Drusen, Stoffwechselendprodukten wie Lipofuszinen sowie einer gestörten Durchblutung der Aderhaut, die in einer sogenannten geographischen Atrophie resultieren kann. Sie kann von der trockenen in die feuchte Form übergehen, bei der es zur Gefäßneubildung, Ödemen und Einblutung in die Netzhaut kommen kann. Die beiden Formen können im fortgeschrittenen Stadium in einen flächigen Zelltod des retinalen Pigmentepitels übergehen.

Die diabetische Retinopathie (DR) ist eine durch die Zuckerkrankheit Diabetes mellitus hervorgerufene Erkankung der Netzhaut des Auges. Die zunehmende Schädigung kleiner Blutgefäße verursacht eine zunächst unbemerkte Schädigung der Netzhaut. Im weiteren Verlauf kann es auch hier zu Ödemen und Einblutung bis hin zur Netzhautablösung kommen. DR kann im Verlauf zur Erblindung führen.

Die bisher verfügbaren pharmakologischen Therapieansätze gegen neurodegenerative Erkrankungen, insbesondere solche des Auges, sind bislang nicht zufriedenstellend.

So haben beispielsweise die zur Behandlung von degenerativen Augenerkrankungen vorgesehenen Substanzen "Ranibizumab" bzw. "Lucentis" sowie "VEGF-Trap" ein in der Praxis begrenztes Wirkungsspektrum. Insbesondere bei erblichen Netzhauterkrankungen sowie der trockenen Form der AMD wirken diese Substanzen nicht oder nicht zufriedenstellend

Die Gruppe der sogenannten VCP-Inhibitoren werden im Stand der Technik zur Behandlung von neurodegenerativen Erkrankungen beschrieben. VCP steht für "Valosin-containing protein" bzw. "Valosin-haltiges Protein". VCP, das auch als p97 bezeichnet wird, ist ein Protein, welches als ein solches beschrieben wird, das in eukaryotischen Zellen in die Proteinqualitätskontrolle und Entfernung von fehlgefalteten Proteinen involviert ist.

In der WO 2013/068431 wird die Behandlung von Muskelschwund aufgrund mutierter und fehlgefalteter SOD1 mittels der VCP-Inhibitoren Eer-I, DBEQ und Syk-Inhibitor-III beschrieben.

Griciuc et al. (2010), Inactivation of VCP/ter94 suppresses retinal pathology caused by misfolded Rhodopsin in Drosophila, PLoS Genet. 6(8): e1001075, beschreiben die Unterdrückung von retinaler Degeneration, verursacht durch fehlgefaltetes Rhodopsin, durch eine Inhibition von VCP.

Valle et al. (2011), Critical role of VCP/p97 in the pathogenesis and progression of non-small cell lung carcinoma, PLoS ONE 6(12): e209073, beschreiben die Hemmung der Lungentumorzellproliferation und -migration und Induktion von Apoptose durch eine Inhibition von VCP.

Muraoka et al. (2013), A novel Valosin-containing protein inhibitor suppresses photoreceptor degeneration in a rapid model of Retinitis pigmentosa, Poster Session ARVO Jahreskonferenz Seattle 2013, beschreiben die Unterdrückung der Photorezeptordegeneration, verursacht durch missgefaltetes Rhodopsin, durch den Einsatz von VCP-Inhibitoren.

In der EP 2 623 494 werden verschiedene VCP-Inhibitoren zur Behandlung von Glaukomen und Retinitis pigmentosa vorgeschlagen, die auf Proteinfaltungsstörungen beruhen.

In dem Dokument WO 2009/011910 wird ein VCP-Inhibitor mit der Bezeichnung "Eeyarestatin" beschrieben. Chou et al. (2013), ChemMedChem 8, 297 ― 312, beschreiben Struktur-Aktivitäts-Experimente, die an den beiden VCP-Inhibitoren ML240 und ML241 im Hinblick auf das inhibitorische Potenzial auf die p97-ATPase durchgeführt wurden.

Zufriedenstellende pharmakologische Interventionen zur Behandlung von solchen neurodegenerativen Erkrankungen, die nicht auf einer Proteinfaltungsstörung beruhen, stehen im Stand der Technik bislang nicht zur Verfügung.

Vor diesem Hintergrund ist es eine Aufgabe der vorliegenden Erfindung eine Substanz bereitzustellen, die wirksam in der Prophylaxe und/oder Behandlung einer nicht auf einer Proteinfaltungsstörung beruhenden neurodegenerativen Augenerkrankung ist.

Diese Aufgabe wird durch die Bereitstellung bzw. Verwendung eines Inhibitors des Valosin-haltigen Proteins (VCP-Inhibitor) gelöst, wobei es sich bei der Augenerkrankung um altersbedingte Makuladegeneration (AMD) oder diabetische Retinopathie (DR) handelt.

Diese Erkenntnis war überraschend und so nicht zu erwarten. So ist im Stand der Technik beschrieben, dass VCP-Inhibitoren bei neurodegenerativen Erkrankungen eingesetzt werden können, die auf einer Proteinfaltungsstörung, beispielsweise auf fehlgefaltetem Rhodopsin beruhen.

Die Erkenntnis der Erfinder war auch deshalb nicht zu erwarten, da VCP als ein solches Protein beschrieben ist, das direkt in die Proteinfaltungsmaschinerie der Zelle involviert ist. Bei VCP handelt es sich nach der Literatur um ein ATP-getriebenes Chaperon, das kritische Schritte in der Ubiquitin-abhängigen Proteinqualitätskontrolle regelt, fehlgefaltete Proteine aus dem endoplasmatischen Retikulum extrahiert und diese der proteasolmalen Degradation zuführt. In der Literatur ist ferner bereits beschrieben worden, dass VCP mit fehlgefaltetem Rhodopsin kolokalisiert.

Es wurde deshalb bislang vermutet, dass VCP-Inhibitoren bei neurodegenerativen Erkrankungen, die nicht auf einer Proteinfaltungsstörung beruhen, keinerlei Wirkung zeigen.

Erfindungsgemäß wird unter einer "nicht auf einer Proteinfaltungsstörung beruhenden neurodegenerativen Erkrankung" eine solche Neurodegeneration verstanden, die durch keinerlei Mutation verursacht wird, welche dann in einer Proteinfaltungsstörung resultieren würde. Erfindungsgemäß betrifft dies die altersbedingte Makuladegeneration (AMD), die bei fortschreitendem Altern und/oder aufgrund von entzündlichen Ereignissen auftritt, und die diabetische Retinopathie (DR), die auf einer gestörter zellulärer Homöostase und Sauerstoffversorgung beruht.

Erfindungsgemäß bedeutet "Stabilisierung von Photorezeptoren" das Entgegenwirken einer Degeneration des Photorezeptors, sei es durch eine Verhinderung der Degeneration oder Reduzierung der Degenerationsgeschwindigkeit.

Die Erfinder konnten anhand einer repräsentativen Anzahl verschiedener VCP-Inhibitoren, die chemisch und strukturell unterschiedlich waren, überraschenderweise feststellen, dass Photorezeptoren, die aus Wildtyp-Ratten gewonnen wurden, in Zellkultur nach Behandlung mit VCP-Inhibitoren signifikant länger funktionsfähig bleiben als unbehandelte Photorezeptoren. Die üblicherweise in Zellkulturen beobachtete Degeneration der Photorezeptoren wird durch die Behandlung mit VCP-Inhibitoren zeitlich signifikant verlangsamt. Die Photorezeptoren werden überraschenderweise durch die VCP-Inhibitoren stabilisiert.

Die der Erfindung zugrundeliegende Aufgabe wird hiermit vollkommen gelöst.

Bei der neurodegenerativen Erkrankung handelt es sich um eine Augenerkrankung, die nicht auf einer Proteinfaltungsstörung eines Sehpigments, bspw. von Rhodopsin und/oder Opsin beruht.

Diese Maßnahme hat den Vorteil, dass ein neues Behandlungskonzept für eine wichtige Gruppe von neurodegenerativen Erkrankungen bereitgestellt wird. So basiert der weitaus größte Anteil von Rhetinitis pigmentosa (RP) nicht auf einer Proteinfaltungsstörung des Rhodopsins oder eines anderen Sehpigments bzw. -proteins, sondern geht auf andere Ursachen zurück.

Die Augenerkrankung ist altersbedingte Makuladegeneration (AMD), insbesondere in ihrer trockenen Form, und diabetische Retinophathie (DR).

Für die vorstehend genannten Augenerkrankungen, die nicht auf einer Proteinfaltungsstörung beruhen, stehen im Stand der Technik bislang keine oder nur begrenzt zufriedenstellende Therapiemöglichkeiten zur Verfügung. Die Erfinder stellen hier erstmals ein neues und vielversprechendes Therapiekonzept bereit.

Dabei ist es nach einer bevorzugten Weiterentwicklung bevorzugt, wenn der VCP-Inhibitor intraokular, periokular oder systemisch verabreicht wird.

Diese Maßnahme hat den Vorteil, dass nach Art der neurodegenerativen Erkrankung jeweils die optimale Verabreichungsform gewählt wird.

Bei der offenbarten Verwendung des VCP-Inhibitors zur Stabilisierung von Photorezeptoren liegen die im Photorezeptor lokalisierten Proteine vorzugsweise im Wildtyp vor, wobei es sich bei den im Photorezeptor lokalisierten Proteinen weiter bevorzugt um Sehpigmente und höchst bevorzugt um Rhodopsin und/oder Opsin handelt.

Diese Maßnahme hat den Vorteil, dass nun erstmals mit dem VCP-Inhibitor auch solche Photorezeptoren stabilisiert werden können, die nicht durch eine Mutation in einem Protein des Photorezeptors, wie einem Sehfarbstoff, bspw. Rhodopsin oder Opsin, geschädigt sind und dadurch einer Degeneration unterliegen. Die Stabilisierung erfolgt vielmehr auch in solchen Fällen, in denen die Degeneration durch andere Ursachen wie zunehmendes Alter, gestörte zelluläre Homöostase oder gestörte Sauerstoffzufuhr sowie Entzündung etc. verursacht wird.

Nach einer bevorzugten Ausgestaltung der offenbarten Verwendung des VCP-Inhibitors zur Stabilisierung von Photorezeptoren weist zumindest eines der im Photorezeptor lokalisierten Proteine, wie einem Sehfarbstoff, bspw. Rhodopsin oder Opsin, zumindest eine Mutation oder genetische Risikovariante auf, die nicht in einer Proteinfaltungsstörung resultiert.

Diese Maßnahme hat den Vorteil, dass nunmehr auch die große Gruppe der in Pathologien des Auges auftretenden Phänomene erfasst werden, in denen eine Degenration des Pigmentepithels bzw. des Photorezeptors beobachtet wird, die involvierten oder verursachenden Mutationen oder genetischen Risikovarianten wie bspw. Polymorphismen jedoch nicht zu einer Proteinfaltungsstörung sondern ggf. zu Funktionsstörungen des betroffenen Proteins führen.

Nach einer bevorzugten Weiterentwicklung der offenbarten Verwendung des VCP-Inhibitors zur Stabilisierung von Photorezeptoren umfassen die im Photorezeptor lokalisierten Proteine Sehpigmente, weiter bevorzugt Rhodopsin und Opsin.

Diese Maßnahme hat den Vorteil, dass der VCP-Inhibitor auch zur Stabilisierung solcher Photorezeptoren eingesetzt werden kann, bei denen die wichtigsten Photorezeptorproteine im Wildtyp vorliegen oder eine solche Mutation aufweisen, die zur Photorezeptordegeneration führt, nicht jedoch eine Faltungsstörung des Rhodopsins verursacht.

Die vorliegende Erfindung betrifft ein Inhibitor des Valosin-haltigen Proteins (VCP-Inhibitor) zur Prophylaxe und/oder Behandlung einer nicht auf einer Proteinfaltungsstörung beruhenden neurodegenerativen Augenerkrankung mit den Merkmalen des Patentanspruchs 1.

Vor diesem Hintergrund ist ein weiterer Gegenstand der vorliegenden Erfindung eine pharmazeutische Zusammensetzung zur Prophylaxe und/oder Behandlung einer nicht auf einer Proteinfaltungsstörung beruhenden neurodegenerativen Augenerkrankung, die als Wirkstoff einen VCP-Inhibitor aufweist, wobei die Augenerkrankung altersbedingte Makuladegeneration (AMD) oder diabetische Retinopathie (DR) ist. Dabei kann es sich bei dem VCP-Inhibitor um den alleinigen Wirkstoff handeln. Es können jedoch weitere Wirkstoffe vorgesehen werden, die gegebenenfalls synergistische Effekte induzieren.

Die erfindungsgemäße pharmazeutische Zusammensetzung ist vorzugsweise zur intraokularen, periokularen oder systemischen Verabreichung ausgestaltet. Es kann sich beispielsweise um Augentropfen oder aber eine Injektionslösung oder eine andere pharmazeutische Zusammensetzung handeln, die zusätzlich einen pharmazeutisch akzeptablen Träger sowie Salze, Puffer und weitere Substanzen enthalten können.

Die Merkmale, Vorteile, Eigenschaften und Weiterentwicklungen der erfindungsgemäßen Verwendung gelten für die erfindungsgemäße pharmazeutische Zusammensetzung gleichermaßen.

Offenbart wird ein Verfahren zur Herstellung einer pharmazeutischen Zusammensetzung zur Prophylaxe und/oder Behandlung einer nicht auf einer Proteinfaltungsstörung beruhenden neurodegenerativen Augenerkrankung, wobei die Augenerkrankung altersbedingte Makuladegeneration (AMD) oder diabetische Retinopathie (DR) ist, das die Formulierung eines VCP-Inhibitors in einen pharmazeutisch akzeptablen Träger aufweist.

Pharmazeutisch akzeptable Träger sind im Stand der Technik umfassend beschrieben und richten sich nach der beabsichtigten Applikationsform. Eine Übersicht findet sich beispielsweise in A. Kibbe, "Handbook of Pharmaceutical Excipients", 3rd Edition, 2000, American Pharmaceutical Association and Pharmaceutical Press.

Ein weiterer Aspekt der vorliegenden Offenbarung betrifft ein Verfahren zur Prophylaxe und/oder Behandlung einer nicht auf einer Proteinfaltungsstörung beruhenden neurodegenerativen Erkrankung, das die Verabreichung eines VCP-Inhibitors bzw. der erfindungsgemäßen pharmazeutischen Zusammensetzung in ein Lebewesen umfasst, vorzugsweise in oder auf das Auge des Lebewesens.

Die Merkmale, Eigenschaften, Vorteile und Weiterentwicklungen der erfindungsgemäßen Verwendung gelten für die Verfahren entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen weiter erläutert, aus denen sich weitere Eigenschaften, Merkmale und Vorteile der Erfindung ergeben. Die Ausführungsbeispiele sind rein illustrativ und schränken die Reichweite der vorliegenden Erfindung nicht ein. Dabei wird Bezug genommen auf die beigefügten Figuren, in denen Folgendes dargestellt ist:

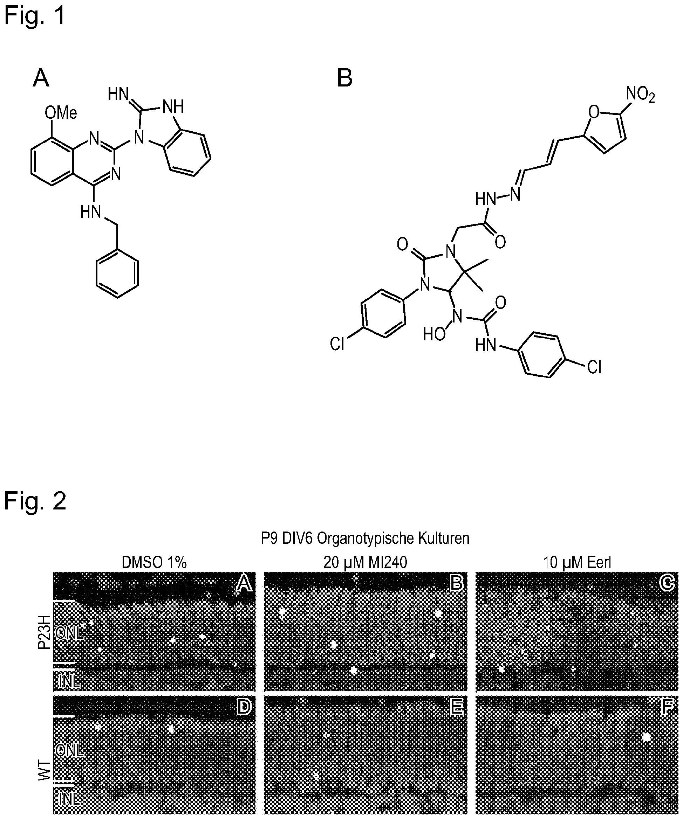

- Fig. 1: zeigt die chemischen Strukturen der VCP-Inhibitoren ML240 (A) und Eeya-restatin I (Eerl) (B).
- Fig. 2: zeigt fluoreszensmikroskopische Aufnahmen eines TUNEL-Assays für sterbende Zellen. Retinale Organkulturen, die von in Rhodopsin mutierten (P23H) und Wildtypratten erhalten wurden, wurden mit ML240 (B und E) und Eerl (C und F) behandelt. Die Kontrollen wurden mit DMSO behandelt. Von den Kulturen wurden Radialschnitte angefertigt und eine TUNEL-Färbung durchgeführt.

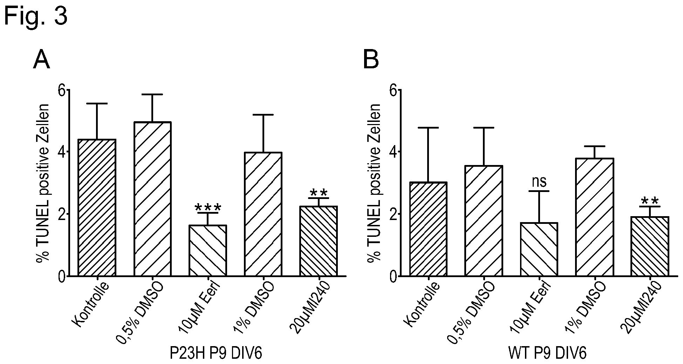

- Fig. 3: zeigt in einem Balkendiagramm das Ergebnis aus Fig.2, d.h. den Photorezeptorzelltod in kultivierten Retinas, die aus in Rhodopsin mutierten (P23H) (A) und Wildtypratten (B) gewonnen wurden, in Abhängigkeit der VCP-Inhibitoren ML240 und Eerl. Dargestellt ist der prozentuale Anteil von TUNEL-positiven Zellen in der äußeren nukleären Schicht (ONL) im Vergleich zu der Gesamtzahl der Zellkerne in der ONL.

### Ausführungsbeispiele

### Material und Methoden

### Ethik

Sämtliche Maßnahmen wurden von dem Tierschutzkomitee der Universität Tübingen genehmigt und in Übereinstimmung mit der Erklärung der Association for Research in Vision and Ophthalmology ("AFO Statement") durchgeführt. Die Protokolle stehen im Einklang mit § 4 Abs. 3 des Deutschen Tierschutzgesetzes und wurden durch die "Einrichtung für Tierschutz, tierärztlichen Dienst und Labortierkunde" geprüft und genehmigt. Es wurden jegliche Bemühungen unternommen, um die Anzahl der Tiere und deren Leid zu minimieren.

### Tiere

Homozygote P23H Rhodopsin-transgene Ratten (hergestellt von Chrysalis DNX Transgenic Sciences, Princeton, NJ, Vereinigte Staaten von Amerika) der Linie Tg (P23H) 1Lav (P23H-1) wurden freundlicherweise bereitgestellt von Dr. M.M. LaVail (Universität von Kalifornien, San Francisco, CA, Vereinigte Staaten von Amerika) und in den Tierbehausungseinrichtungen des Zentrums für Ophthalmologie in Tübingen gehalten. Es wurden heterozygote P23H-Ratten verwendet, die durch Kreuzung mit wt, CD Ratten (CDH IGS Rat; Charles River, Deutschland) erhalten wurden, um den genetischen Hintergrund der autosomal-dominanten Retinitis pigmentosa (ADRP) wieder zu geben.

### Versuchstiere

Retinas vom postnatalen Tag 9 (PN9) von P23H-transgenen Ratten wurden für sechs Tage (DIV6) kultiviert, was PN15, d.h. dem Höhepunkt der Degeneration in solchen Mutanten entspricht, die *in vivo* hinsichtlich des Alters angepasst wurden. Der Tag der Geburt wurde auf P0 festgelegt. Zur Anfertigung von Radialschnitten wurden die Kulturen für 30 Minuten bei 4°C in 4 % Paraformaldehyd in 0,1 M Phosphatpuffer (PB; pH 7,4) getränkt und in Cryomatrix (Tissue-Tek, Leica, Bensheim, Deutschland) eingebettet. Die Radialschnitte (15 µm Dicke) wurden sofort verarbeitet oder bei 20°C gelagert.

### Präparation von Organkulturen

Die Retinas wurden von neun Tage alten P23H-Ratten mit dem anhaftendem retinalen Pigmentepithel (RPE) isoliert, im Wesentlichen wie zuvor beschrieben in Caffe et al. (2001), "Mouse retina explants after long-term culture in serum free medium", J. Chem. Neuroanat. 22(4): 263-73, und Arango-Gonzalez et al. (2010), "In vivo and in vitro development of S- and M-cones in rat retina", Invest. Ophthalmol. Vis. Sci. 2010 Oct;51(10):5320-7. In Kürze, die Augen von enthaupteten Ratten wurden für 15 Minuten bei 37°C in einer aseptischen Umgebung entkernt und mit 12 % Proteinase K (ICN Biomedicals Inc., OH, Vereinigte Staaten von Amerika) in R16 serumfreien Kulturmedium (Invitrogen Life Technologies, Paisley, Vereinigtes Königreich) vorbehandelt. Der enzymatische Verdau wurde durch Zugabe von 20 % fötalem Rinderserum gestoppt. Retina und RPE wurden freigelegt und vier Radialschnitte wurden getätigt, um das Gewebe flach zu machen. Das Gewebe wurde auf eine 0,5 µm Polycarbonatmembran (Corning Life Sciences, Lowell, MA, Vereinigte Staaten von Amerika) transferiert, wobei das RPE der Membran zugewandt war. Die Inserts wurden in Kulturplatten mit sechs Vertiefungen gegeben und mit R16-Nährmedium bei 36,5°C versetzt. Die Retina wurde in R16 Kulturmedium ohne Behandlung für einen Tag belassen, um eine Gewöhnung an die Kulturbedingungen zu ermöglichen. Das Medium wurde bei DIV1, DIV3 und DIV5 ausgetauscht und neues Medium und enthielt entweder ML240 (1 µM oder 20 µM) oder Eerll (1 µM oder 20 µM). Beide Verbindungen wurden vorab in Dimethylsulfoxid (DMSO, Sigma) verdünnt. Als Kontrollen wurde die gleiche Menge von DMSO (0,05 % bzw. 1 %) in Kulturmedium verdünnt. Die Kulturen wurden bei DIV6 fixiert und als Radialschnitte analysiert.

### TUNEL-Assay

Ein TUNEL-Assay ("terminal deoxynucleotidyltransferase dUTP nick end labeling") wurde durchgeführt, indem ein *in situ* Zelltoddetektionskit konjugiert mit Fluoreszeinisothiocyanat (Roche Diagnostics, Mannheim, Deutschland) verwendet wurde.

### Immunhistochemie

Die Schnitte wurden über Nacht bei 4°C mit primären Antikörpern gegen Rhodopsin oder Visual Arrestin inkubiert. Die Fluoreszenzimmuncytochemie wurde unter Verwendung von mit Alexa Fluor^{®} 488 und 564 konjugierten Sekundärantikörpern (Molecular Probes, Incorporation, Eugene, Vereinigte Staaten von Amerika) durchgeführt. Negativkontrollen wurden durchgeführt, indem der Primärantikörper weggelassen wurde.

### Ergebnisse

Retinale Organkulturen wurden aus in Rhodopsin mutierten (P23H) Ratten und aus Wildtypratten gewonnen. Die P23H-Mutation im Rhodopsin der Ratte entspricht der P37H-Mutation beim Menschen (Rh1^{P37H}), die zu fehlgefaltetem Rhodamin und einer Form von RP führt. Diese Organkulturen wurden mit den VCP-Inhibitoren ML240 (Fig. 1A) und Eerl (Fig. 1B) behandelt. Die hiervon angefertigten Radialschnitte zeigen einen deutlichen Anstieg des Überleben solcher Photorezeptoren und eine reduzierte Tunnelfärbung solcher Retinas, die mit ML240 (Fig. 2; B und E) und Eerl (Fig. 2; C und F) behandelt wurden, im Vergleich zur Kontrolle, die nur mit DMSO behandelt wurde (Fig. 2; A und D).

Dabei zeigt sich erwartungsgemäß, dass in der P23H Mutante beide VCP-Inhibitoren, also sowohl Eerl als auch ML240, die Anzahl von sterbenden Photorezeptoren im Vergleich zu den entsprechend unbehandelten und DMSO-behandelten (0,5 und 1 %) Kontrollen signifikant reduziert (Fig. 3; A). Überraschenderweise reduzieren auch in WT-kultivierten Retinas die VCP-Inhibitoren den prozentualen Anteil von TUNEL-positiven Zellen, ML240 signifikant und Eerl nicht signifikant (Fig. 3; B); **p < 0,01; ***p < 0,001. Die VCP-Inhibitoren haben also auch auf "gesunde" Photorezeptoren, die in ihren Proteinen keine Mutation aufweisen, eine stabilisierende Wirkung.

### Fazit

Die Erfinder konnten auf eindrucksvolle Weise anhand eines Retinaexplantatsystems demonstrieren, dass VCP-Inhibitoren eine Stabilisierung auf Photorezeptoren ausüben, deren Proteine im Wildtyp bzw. nicht faltungsmutiert vorliegen. Dies führt zur erfinderischen Erkenntnis, dass VCP-Inhibitoren zur Prophylaxe und/oder Behandlung einer nicht auf einer Proteinfaltungsstörung beruhenden neurodegenerativen Erkrankung und zur Stabilisierung von Photorezeptoren verwendet werden können.

## Patentansprüche

1. Inhibitor des Valosin-haltigen Proteins (VCP-Inhibitor) zur Verwendung in der Prophylaxe und/oder Behandlung einer nicht auf einer Proteinfaltungsstörung beruhenden neurodegenerativen Erkrankung, wobei es sich um eine Augenerkrankung handelt, **dadurch gekennzeichnet, dass** die Augenerkrankung altersbedingte Makuladegeneration (AMD) oder diabetische Retinopathie (DR) ist.

2. VCP-Inhibitor nach Anspruch 1, **dadurch gekennzeichnet, dass** es sich bei der AMD um die in ihrer trockenen Form handelt.

3. VCP-Inhibitor nach Anspruch 1 oder 2, **dadurch gekennzeichnet, dass** der VCP-Inhibitor in einer Augentropflösung, als intraokular oder systemisch verabreichtes Präparat oder als Nahrungsergänzungsmittel vorliegt.

4. Pharmazeutische Zusammensetzung zur Verwendung in der Prophylaxe und/oder Behandlung einer nicht auf einer Proteinfaltungsstörung beruhenden neurodegenerativen Erkrankung, die als Wirkstoff den VCP-Inhibitor nach einem der Ansprüche 1 bis 3 aufweist, wobei es sich bei der nicht auf einer Proteinfaltungsstörung beruhenden neurodegenerativen Erkrankung um die Augenkrankheit der altersbedingten Makuladegeneration (AMD) oder diabetische Retinopathie (DR) handelt.

5. Pharmazeutische Zusammensetzung nach Anspruch 4, **dadurch gekennzeichnet dass** diese als Augentropflösung, als intraokular oder systemisch verabreichtes Präparat oder als Nahrungsergänzungsmittel ausgestaltet ist.

## Claims

1. An inhibitor of the valosin-containing protein (VCP inhibitor) for use in the prophylaxis and/or treatment of a neurodegenerative disease not based on a protein folding disorder, wherein the disease is an eye disease, **characterized in that** the eye disease is age-related macular degeneration (AMD) or diabetic retinopathy (DR).

2. The VCP inhibitor according to claim 1, **characterized in that** the AMD is in its dry form.

3. The VCP inhibitor according to claim 1 or 2, **characterized in that** the VCP inhibitor is present in an eye drop solution, as an intraocularly or systemically administered preparation or as a food supplement.

4. A pharmaceutical composition for use in the prophylaxis and/or treatment of a neurodegenerative disease not based on a protein folding disorder, which comprises the VCP inhibitor according to any one of claims 1 to 3 as an active ingredient, the neurodegenerative disease not being based on a protein folding disorder is the eye disease of age-related macular degeneration (AMD) or diabetic retinopathy (DR).

5. The pharmaceutical composition according to claim 4, **characterized in that** it is configured as an eye drop solution, as an intraocularly or systemically administered preparation or as a food supplement.

## Revendications

1. Inhibiteur de la protéine contenant de la valosine (inhibiteur de VCP) destiné à être utilisé dans la prophylaxie et/ou le traitement d'une maladie neurodégénérative qui n'est pas due à un trouble du repliement des protéines, dans lequel il s'agit d'une maladie oculaire, **caractérisé en ce que** la maladie oculaire est une dégénérescence maculaire liée à l'âge (DMLA) ou une rétinopathie diabétique (RD).

2. Inhibiteur de VCP selon la revendication 1, **caractérisé en ce que** la DMLA est la DMLA sèche.

3. Inhibiteur de VCP selon la revendication 1 ou 2, **caractérisé en ce que** l'inhibiteur de VCP se présente dans un collyre en tant que préparation administrée par voie intra-oculaire ou par voie systémique ou en tant que complément alimentaire.

4. Composition pharmaceutique destinée à être utilisée dans la prophylaxie et/ou dans le traitement d'une maladie neurodégénérative qui n'est pas due à un trouble du repliement des protéines, qui présente en tant que principe actif l'inhibiteur de VCP selon l'une quelconque des revendications 1 à 3, dans laquelle la maladie neurodégénérative qui n'est pas due à un trouble du repliement des protéines est la maladie oculaire de la dégénérescence maculaire liée à l'âge (DMLA) ou la rétinopathie diabétique (RD).

5. Composition pharmaceutique selon la revendication 4, **caractérisée en ce qu'**elle est conçue en tant que collyre, en tant que préparation administrée par voie intra-oculaire ou par voie systémique ou en tant que complément alimentaire.
